# FASCICULE DE BREVET EUROPEEN

(11) **EP 0 605 320 B1**
(45) Date de publication et mention de la délivrance du brevet: **09.04.1997**
(21) Numéro de dépôt: 93403190.7
(22) Date de dépôt: 28.12.1993
(51) Int. Cl.: A61K 7/13

(54) **Compositions tinctoriales pour fibres kératiniques à base de paraphénylènediamines, de métaphénylènediamines et de dérivés du benzimidazole, et procédé de teinture les mettant en oeuvre**
Färbemittel für keratinische Fasern enhaltend Paraphenylenediamines, Metaphenylenediamines und Benzimidazolderivaten, sowie Färbungsverfahren unter deren Verwendung
Dye composition for keratinic fibres based on paraphenylenediamine, metaphenylenediamines and benzimidazol derivates, and the dyeing process using same

(30) Priorité: 30.12.1992 FR 9215945
(43) Date de publication de la demande: 06.07.1994
(73) Titulaire: L'OREAL, 75008 Paris (FR)
(72) Inventeur: Audousset, Marie-Pascale, F-92300 Levallois-Perret (FR); Cotteret, Jean, F-78480 Verneuil-sur-Seine (FR)
(74) Mandataire: Casalonga, Axel

(56) Documents cités:
- EP-A- 0 004 368
- EP-A- 0 467 026
- DE-A- 1 921 911
- GB-A- 2 205 111

## Description

La présente invention est relative à de nouvelles compositions tinctoriales pour fibres kératiniques et en particulier pour cheveux humains, contenant des précurseurs de colorants d'oxydation et des coupleurs dérivés du benzimidazole et des métaphénylènediamines et aux procédés de teinture mettant en oeuvre de telles compositions.

Il est connu de teindre les fibres kératiniques et en particulier les cheveux humains, avec des compositions tinctoriales contenant des précurseurs de colorants d'oxydation et en particulier des paraphénylènediamines, des ortho- ou des para-aminophénols appelés généralement "bases d'oxydation".

On sait également qu'on peut faire varier les nuances obtenues avec ces bases d'oxydation en les associant à des coupleurs encore appelés modificateurs de coloration, choisis notamment parmi les métadiamines aromatiques, les méta-aminophénols, les métadiphénols.

Les métadiamines aromatiques sont connues en particulier pour permettre l'obtention de nuances bleues ou grises avec les paraphénylènediamines.

Le brevet français FR-A-2.013.346 décrit l'utilisation comme coupleur de dérivés du benzimidazole permettant l'obtention de gammes de coloration allant du blond au bleu et au gris.

Dans le brevet allemand DE-A-2.812.678, on décrit également l'utilisation d'autres dérivés de benzimidazole en mentionnant leurs propriétés tinctoriales.

On recherche dans le domaine de la coloration capillaire, des précurseurs de colorants d'oxydation ou des coupleurs permettant de conférer aux cheveux une coloration ayant une résistance satisfaisante à la lumière, aux lavages, aux intempéries et à la transpiration.

On est plus particulièrement attentif à avoir des colorations présentant de très bons comportements à des alternances d'irradiations et de lavages.

Les compositions de l'état de la technique précitées ne donnent pas totalement satisfaction sur ce plan, notamment en ce qui concerne la réalisation de nuances cendrées ou bleues.

La demanderesse a découvert de manière surprenante que l'utilisation d'un coupleur dérivé du benzimidazole associé à des métaphénylènediamines et à des bases d'oxydation de la famille des dérivés para et/ou ortho, conduisait à des colorations présentant une ténacité particulièrement remarquable, notamment lorsque les fibres kératiniques teintes et en particulier les cheveux humains sont soumis à des expositions alternées à des irradiations, notamment aux rayons UV, et à des lavages répétés.

L'invention a donc pour objet des compositions tinctoriales destinées à être utilisées pour la teinture d'oxydation des fibres kératiniques, et en particulier des cheveux humains, contenant des précurseurs de colorants d'oxydation de type para et/ou ortho et des coupleurs constitués par au moins un dérivé de benzimidazole et au moins une métaphénylènediamine.

L'invention a également pour objet l'utilisation du benzimidazole comme coupleur dans une composition tinctoriale d'oxydation, contenant un précurseur de colorant d'oxydation para et/ou ortho et une métaphénylènediamine.

Un autre objet de l'invention est constitué par le procédé de coloration des fibres kératiniques et en particulier des cheveux humains, mettant en oeuvre de telles compositions en présence d'un agent oxydant.

D'autres objets de l'invention apparaîtront à la lecture de la description et des exemples qui suivent.

Les dérivés de benzimidazole utilisés comme coupleurs pour la teinture d'oxydation des fibres kératiniques et en particulier des cheveux humains, en présence d'au moins une métaphénylènediamine et d'au moins un précurseur de colorant d'oxydation para et/ou ortho, répondent à la formule (I) : 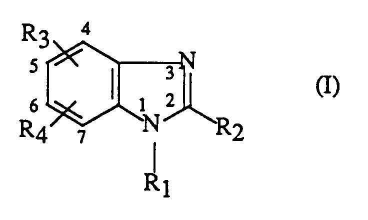 dans laquelle :
R₁ présente un atome d'hydrogène ou un radical alkyle en C₁-C₄ ;
R₂ représente un atome d'hydrogène ou un radical alkyle en C₁-C₄ ou un radical phényle;
R₃ représente un radical hydroxyle, amino ou méthoxy;
R₄ représente un atome d'hydrogène, un radical hydroxyle ou méthoxy ou alkyle en C₁-C₄;
   sous réserve que :
   - lorsque R₃ désigne NH₂, il occupe la position 4;
   - lorsque R₃ est en position 4, R₄ est en position 7;
   - lorsque R₃ est en position 5, alors R₄ est en position 6.

Les benzimidazoles plus particulièrement utilisables sont le 4-hydroxybenzimidazole, le 4-aminobenzimidazole, le 4-hydroxy 7-méthylbenzimidazole, le 2-méthyl 4-hydroxybenzimidazole, le 1-butyl 4-hydroxybenzimidazole, le 2-méthyl 4-aminobenzimidazole, le 5,6-dihydroxybenzimidazole, le 5-hydroxy 6-méthoxybenzimidazole, le 4,7-dihydroxybenzimidazole, le 4,7-dihydroxy 1-méthylbenzimidazole, le 4,7-diméthoxybenzimidazole, le 5,6-dihydroxy 1-méthylbenzimidazole, le 5,6-dihydroxy 2-méthylbenzimidazole, le 5,6-diméthoxybenzimidazole.

Le 4-hydroxybenzimidazole est plus particulièrement préféré.

Les précurseurs de colorants de type para ou ortho sont des composés qui ne sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux groupements amino, soit un groupement amino et un groupement hydroxy, en position para ou ortho, l'un par rapport à l'autre.

Les précurseurs de colorants d'oxydation de type para sont choisis plus particulièrement parmi les paraphénylènediamines, les paraaminophénols, les précurseurs hétérocycliques para, comme la 2,5-diaminopyridine, la 2-hydroxy 5-aminopyridine, des tétraaminopyri midine et les bases dites "doubles" de la famille des bis-phényl alkylènediamines.

A titre de paraphénylènediamines, on peut citer les composés répondant à la formule (II) ci-après : 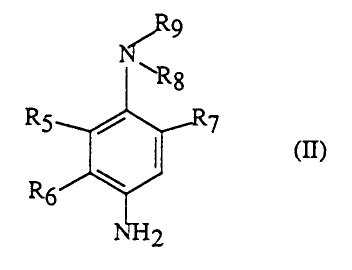 dans laquelle :
R₅, R₆ et R₇, identiques ou différents, représentent un atome d'hydrogène ou d'halogène, un radical alkyle ou hydroxyalkyle ayant 1 à 4 atomes de carbone, un radical alcoxy ayant 1 à 4 atomes de carbone, un radical carboxy ou sulfo;
R₈ et R₉, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ces groupes alkyle ou alcoxy ayant de 1 à 4 atomes de carbone, ou bien R₈ et R₉ forment, conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés.

Parmi les composés de formule (II), on peut citer la p-phénylène diamine, la p-toluylènediamine, la méthoxyparaphénylènediamine, la chloroparaphénylènediamine, la 2,6-diméthylparaphénylènediamine, la 2,6-diéthylparaphénylènediamine, la 2,5-diméthylparaphénylène diamine, la 2-méthyl 5-méthoxyparaphénylènediamine, la 2,6-diméthyl 5-méthoxyparaphénylènediamine, la N,N-diméthylparaphénylène diamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-((β-hydroxyéthyl) aniline, la 4-amino N,N-(éthylcarbamylméthyl)aniline, la 3-méthyl 4-amino N,N-(éthylcarbamylméthyl)aniline, la 4-amino N,N-(éthyl,β-pipéridinoéthyl)aniline, la 3-méthyl 4-amino N,N-(éthyl,β-pipéndinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl) aniline, la 4-amino N,N-(éthyl,β-mésylaminoéthyl)aniline, la 3-méthyl 4-amino N,N-(éthyl,β-mésylaminoethyl)aniline, la 4-amino N,N-(éthyl, β-sulfoéthyl)aniline, la 3-méthyl 4-amino N,N-(éthyl,β-sulfoéthyl)aniline, la N-[(4'-amino)phényl]morpholine, la N-[(4'-amino) phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol, le 2-β-hydroxyéthylaminométhyl 4-aminophénol.

Les colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène, les orthophénylènediamines.

Les bases dites doubles sont des bis-phénylalkylènediamines, répondant à la formule : 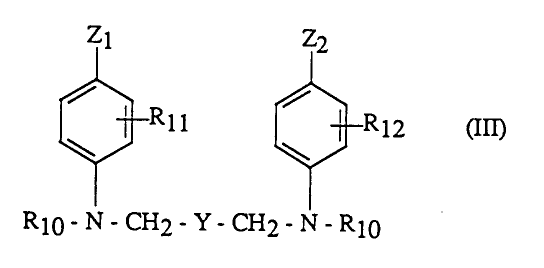 dans laquelle :
Z₁ et Z₂ identiques ou différents, représentent des groupements hydroxyle ou NHR₃, où R₃ désigne un atome d'hydrogène ou un radical alcoyle inférieur;
R₁₁ et R₁₂, identiques ou différents, représentent soit des atomes d'hydrogène, soit des atomes d'halogène, soit encore des groupements alcoyle;
R₁₀ représente un atome d'hydrogène, un groupe alcoyle, hydroxyalcoyle ou aminoalcoyle, dont le reste amino peut être substitué;
Y représente un radical pris dans le groupe constitué par les radicaux suivants : -(CH₂)ₙ-, -(CH₂)ₘ-O-(CH₂)ₘ-, (CH₂)ₘ-CHOH-(CH₂)ₘ-,
n étant un nombre entier compris entre 0 et 8 et m un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'-bis-(β-hydroxyéthyl)N,N'-bis-(4'-aminophényl)1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N, N'-bis-(4-méthylaminophényl)tétraméthylènediamine, la N,N'-bis-(éthyl)N,N'-bis-(4'-amino 3'-méthylphényl)éthylènediamine.

Les métaphénylènediamines qui sont utilisables dans l'invention, répondent à la formule (IV) : 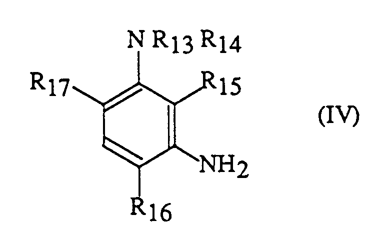 dans laquelle :
R₁₃ et R₁₄ désignent, indépendamment l'un de l'autre, hydrogène, un groupement alkyle en C₁-C₄, hydroxyalkyle en C₁-C₄ ;
R₁₅ désigne hydrogène ou un groupement alkyle ou alcoxy en C₁-C₄ ;
R₁₆ désigne un atome d'hydrogène ou un groupement alkyl(C₁-C₄) ou hydroxyalcoxy en C₁-C₄, ou alcoxy en C₁-C₄;
R₁₇ hydroxyalcoxy un atome d'hydrogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, un atome d'halogène, carboxyalcoxy en C₁-C₄, 2',4'-diaminophénoxyalcoxy en C₁-C₄, aminoalcoxy en C₁-C₄ ;
sous réserve que si R₁₇ désigne carboxyalcoxy ou 2',4'-diaminophénoxyalcoxy, alors R₁₃, R₁₄, R₁₅ et R₁₆ désignent hydrogène.

Ces composés sont utilisables sous forme libre ou salifiée.

Les métaphénylènediamines plus particulièrement préférées et répondant à la formule (IV) sont choisies parmi le 1,3-diaminobenzène, le 1-méthoxy 2,4-diaminobenzène, le 1-(2-hydroxyéthyloxy)2,4-diaminobenzène, le 1-éthoxy 2,4-diaminobenzène, le 1-méthyl 2,6-diaminobenzène, le 1-amino 3-[(N,N-bis-(2-hydroxyéthyl)amino]benzène, le 1-β-hydroxyéthyl 2,4-diaminobenzène, le 1-méthoxy 2-amino 4-β-hydroxyéthylaminobenzène, le 1,3-diamino 4-β-aminoéthyloxybenzène, le 1-(2-hydroxy-éthyloxy)2-amino 4-méthylaminobenzène, l'acide 2,4-diaminophénoxyacétique, le 4,6-bis-(2-hydroxyéthyloxy)1,3-diaminobenzène, le 2,4-diamino 5-méthylphénétol, le 2,4-diamino 5-(β-hydroxyéthyloxy)toluène, le 2,4-diméthoxy 1,3-diaminobenzène.

Les compositions tinctoriales pour fibres kératiniques, en particulier pour cheveux humains, qui constituent un autre objet de l'invention, sont essentiellement caractérisées par le fait qu'elles contiennent dans un milieu cosmétiquement acceptable et approprié pour la teinture des fibres kératiniques, ce milieu étant cosmétique lorsque les compositions sont appliquées sur les cheveux humains, contenant au moins un précurseur de colorant d'oxydation para et/ou ortho, au moins une métaphénylènediamine et au moins un dérivé de benzimidazole répondant à la formule (I).

Les composés plus particulièrement préférés utilisés dans les compositions conformes à l'invention, sont ceux définis ci-dessus.

Une forme de réalisation particulièrement préférée consiste à utiliser dans la même composition le dérivé de benzimidazole de formule (I), au moins une métaphénylènediamine définie ci-dessus, au moins une paraphénylènediamine et au moins une base double de la famille des bis-phénylalkylènediamines.

Conformément à l'invention, les compositions peuvent également contenir d'autres coupleurs habituellement utilisés dans les compositions tinctoriales pour fibres kératiniques, en particulier pour cheveux, tels que plus particulièrement les métadiphénols, les métaaminophénols, les métaacylaminophénols, les métauréidophénols, les métacarbalcoxyaminophénols, les hydroxynaphtols, des coupleurs possédant un groupement méthylène actif, tels que les composés β-cétoniques, les pyrazolones ou les coupleurs indoliques décrits plus particulièrement dans les demandes de brevet et brevets FR-A-2.636.236, EP-A-0428.442, EP-A-0428.441, EP-A-0496.653, EP-A-0424.261, ainsi que le 4-hydroxyindole.

Parmi les coupleurs, on peut plus particulièrement citer le métaaminophénol, le 1,3-dihydroxy 4-chlorobenzène, le 1,3-dihydroxybenzène, l'α-naphtol, le 6-hydroxybenzomorpholine, le 1-méthyl 2-hydroxy 4-aminobenzène, le 1-méthyl 2-hydroxy 4-(2-hydroxyéthyl)aminobenzène, le 1,3-dihydroxy 2-méthylbenzène, le 1-hydroxy 3,4-méthylènedioxybenzène, le 1-(β-hydroxyéthylamino)3,4-méthylènedioxybenzène, le 2-bromo 4,5-méthylènedioxyphénol, le 2-amino 5-acétamidophénol, la 2,6-diaminopyridine, le 6-hydroxyindole, le 7-hydroxyindole, le 7-aminoindole.

Il est possible d'utiliser conjointement avec les précurseurs de colorants d'oxydation et les coupleurs précités, des colorants directs tels que des colorants azoïques, anthraquinoniques ou des dérivés nitrés de la série benzénique, notamment en vue de nuancer ou d'enrichir en reflets les colorations apportées par les précurseurs de colorants d'oxydation.

Le dérivé de benzimidazole de formule (I) et les compositions tinctoriales définies ci-dessus, sont mis en oeuvre conjointement avec un agent oxydant qui peut être introduit dans la composition tout juste avant l'emploi ou appliqué sur les fibres avant, pendant ou après l'application de la composition tinctoriale contenant les précurseurs définis ci-dessus.

A titre d'agent oxydant, on peut plus particulièrement citer le peroxyde d'hydrogène, le peroxyde d'urée, les bromates de métaux alcalins, les persels tels que les perborates et les persulfates, le peroxyde d'hydrogène étant plus particulièrement préféré.

Les compositions tinctoriales prêtes à l'emploi constituent un autre objet de l'invention et sont caractérisées par le fait qu'elles contiennent dans un milieu approprié pour la teinture :
a) au moins un dérivé de la famille des benzimidazoles de formule (I),
b) au moins un précurseur de colorant d'oxydation para et/ou ortho choisi parmi les paraphénylènediamines, les paraaminophénols, les précurseurs hétérocycliques, les orthoaminophénols, les orthophénylènediamines, les bases dites doubles de la famille des bisphénylalkylènediamines,
c) au moins une métaphénylènediamine, et
d) au moins un agent oxydant défini ci-dessus.

Les précurseurs de colorants par oxydation de type para et/ou ortho ainsi que les coupleurs peuvent être introduits dans les compositions conformes à l'invention, soit sous forme de base libre, soit sous forme de sels tels que sous forme de chlorhydrate, de bromhydrate ou de sulfate.

L'ensemble des précurseurs de colorants par oxydation de type para et/ou ortho ainsi que les coupleurs utilisés conformément à l'invention, représente de 0,02 à 10% et de préférence de 0,1 à 10% en poids par rapport au poids total de la composition. La concentration en composés de formule (I) est généralement comprise entre 0,004 et 3,5% et de préférence entre 0,05 et 3,5% en poids par rapport au poids total de la composition. La concentration en métaphénylènediamine est généralement comprise entre 0,004 et 3,5% et de préférence entre 0,01 et 3,5% en poids par rapport au poids total de la composition.

Le rapport entre la métaphénylènediamine et le dérivé de benzimidazole est de préférence compris entre 0,01 et 6,5.

Le pH des compositions appliquées sur les fibres kératiniques et en particulier les cheveux, est généralement compris entre 3 et 11.

Ce pH est ajusté par l'utilisation d'agents acidifiants ou alcalinisants bien connus dans le domaine de la teinture capillaire.

Les compositions tinctoriales conformes à l'invention contiennent généralement des agents tensio-actifs anioniques, cationiques, nonioniques, amphotères ou leurs mélanges, bien connus dans l'état de la technique.

Ces agents tensio-actifs sont présents dans les compositions conformes à l'invention dans des proportions comprises entre 0,5 et 55% en poids et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention, peuvent être choisis parmi l'alginate de sodium, la gomme arabique, les dérivés de cellulose, les polymères d'acide acrylique, la gomme de xanthane. On peut également utiliser des agents épaississants minéraux tels que la bentonite.

Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Conformément à l'invention, le procédé consiste à appliquer sur les fibres kératiniques une composition préparée au moment de l'emploi contenant au moins un coupleur de formule (I), au moins une métaphénylènediamine, au moins un précurseur de colorant d'oxydation de type para et au moins un agent oxydant dans une quantité suffisante pour pouvoir développer une coloration.

On utilise de préférence une solution d'eau oxygénée à 20 volumes. Le mélange obtenu est appliqué sur les cheveux et on laisse poser pendant 10 à 40 minutes, de préférence 15 à 30 minutes, après quoi, on rince les cheveux, on les lave au shampooing, on les rince à nouveau et on les sèche.

Une autre forme de réalisation consiste à appliquer séparément une composition (A) contenant le coupleur benzimidazole de formule (I), la métaphénylènediamine et le précurseur de colorant d'oxydation, puis après rinçage, une composition (B) renfermant l'agent oxydant.

Il est également possible, conformément à l'invention, d'appliquer séparément une composition contenant le précurseur d'oxydation puis, après rinçage, d'appliquer une composition renfermant le coupleur benzimidazole de formule (I), la métaphénylènediamine et l'agent oxydant.

Les conditions de pose et de séchage ou de lavage étant similaires à celles indiquées ci-dessus.

Les exemples qui suivent sont destinés à illustrer l'invention sans pour autant présenter un caractère limitatif.

### EXEMPLES 1 à 3

On procède à la teinture des cheveux en appliquant sur des cheveux naturels gris à 90% de blancs, un mélange extemporané (poids pour poids) de la composition colorante (A) et de la composition oxydante (B).

On laisse agir ce mélange pendant 30 minutes, puis on rince les cheveux, on effectue un shampooing. Après séchage, les cheveux sont teints dans la nuance précisée au bas du tableau.

**Tableau**

| en g | 1 | 2 | 3 |
|---|---|---|---|
| **Composition colorante (A)** | | | |
| 4-hydroxybenzimidazole, HBr | 0,0047 | 1,08 | 0,3 |
| Paraphénylènediamine, 2HCl | 0,0382 | 0,01 | 3,1 |
| 2,6-diméthoxymétaphénylènediamine, 2HCl | 0,0063 | | |
| 4,6-di-(β-hydroxyéthyloxy)métaphénylènediamine, 2HCl | | 1,806 | |
| 1-amino 3-(β-hydroxyéthylamino) 6-méthoxybenzène, 2HCl | | | 0,423 |
| Paratoluènediamine, 2HCl | | 0,92 | |
| Support de coloration (I) | X | X | X |
| Eau qsp | 100 | 100 | 100 |

| **Composition oxydante (B)** | | | |
|---|---|---|---|
| Solution d'eau oxygénée à 20 volumes | | | |
| Acide phosphorique qs pH 3 | 100 | 100 | 100 |
| **Nuances obtenues** : sur cheveux naturels gris 90% blancs | beige léger | bleu intense | brun |

### SUPPORT DE COLORATION (I)

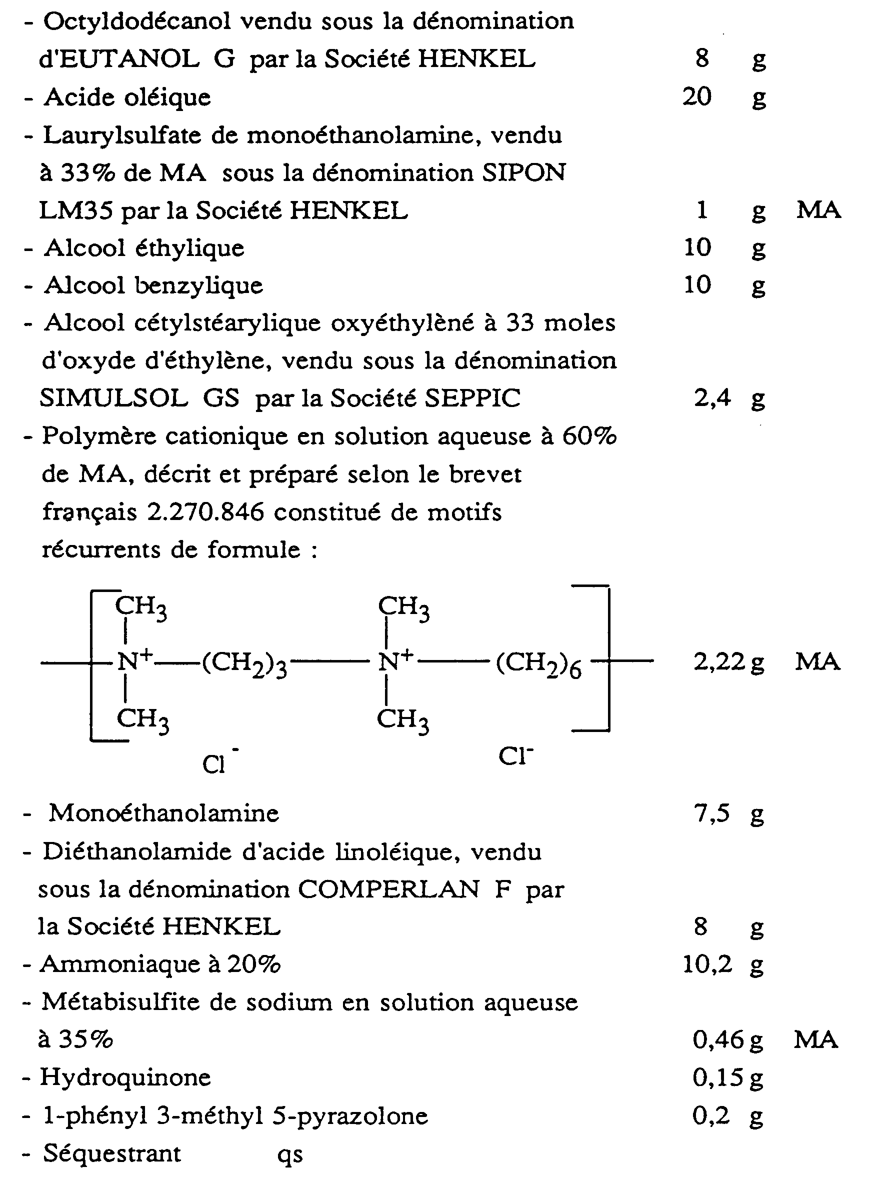

### EXEMPLES 4 et 5

On prépare les compositions suivantes :

**Tableau**

| en g | 4 | 5 |
|---|---|---|
| **Composition colorante (A)** | | |
| 4-hydroxybenzimidazole, HBr | 0,005 | 1,08 |
| Paraphénylènediamine, 2HCl | 0,038 | 0,01 |
| Paratoluylènediamine, 2HCl | | 0,92 |
| 2,4-diaminophénoxyéthanol | 0,006 | 1,46 |
| Support de coloration (II) | X | X |
| Eau qsp | 100 | 100 |

| **Composition oxydante (B)** | | |
|---|---|---|
| Solution d'eau oxygénée à 20 volumes (pH 3) | 100 | 100 |
| **Nuances obtenues** : | blond clair | Noir |

### SUPPORT DE COLORATION (II)

| | |
|---|---|
| - Alcool oléique polyglycérolé à 2 moles de glycérol | 4 g |
| - Alcool oléique polyglycérolé à 4 moles de glycérol (78% de MA) | 5,69 g MA |
| - Acide oléique | 3 g |
| - Amine oléique à 2 moles d'oxyde d'éthylène, vendue sous la dénomination ETHOMEEN 012 par la Société AKZO | 7 g |
| - Laurylaminosuccinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% de MA | 0,455 g MA |
| - Acétate d'ammonium | 0,8 g |
| - Antioxydant, séquestrant qs | |
| - Parfum, conservateur qs | |
| - Ammoniaque à 20% de NH₃ | 10 g |

La composition obtenue est mélangée, poids pour poids, avec de l'eau oxygénée titrant 20 volumes. Le mélange est appliqué sur des cheveux décolorés à raison de 28 g pour 3 g de cheveux, pendant 30 minutes. Les cheveux sont ensuite rincés, lavés avec un shampooing standard et séchés.

La coloration capillaire est évaluée de manière visuelle.

## Revendications

1. Utilisation de dérivés du benzimidazole comme coupleurs pour la coloration d'oydation de fibres kératiniques et en particulier des cheveux humains, en présence d'au moins une métaphénylènediamine et d'au moins un précurseur de colorant d'oxydation para et/ou ortho, le dérivé de benzimidazole répondant à la formule : 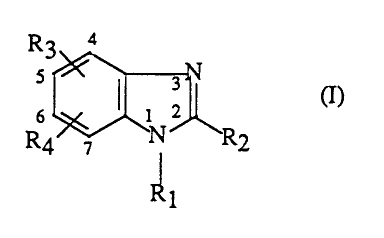 dans laquelle :
R₁ représente un atome d'hydrogène ou un radical alkyle en C₁-C₄ ;
R₂ représente un atome d'hydrogène ou un radical alkyle en C₁-C₄ ou un radical phényle;
R₃ représente un radical hydroxyle, amino ou méthoxy;
R₄ représente un atome d'hydrogène, un radical hydroxyle ou méthoxy ou alkyle en C₁-C₄;
sous réserve que :
- lorsque R₃ désigne NH₂, il occupe la position 4;
- lorsque R₃ est en position 4, R₄ est en position 7;
- lorsque R₃ est en position 5, alors R₄ est en position 6.

2. Utilisation selon la revendication 1, caractérisée par le fait que le dérivé de benzimidazole est choisi parmi le 4-hydroxybenzimidazole, le 4-aminobenzimidazole, le 4-hydroxy 7-méthylbenzimidazole, le 2-méthyl 4-hydroxybenzimidazole, le 1-butyl 4-hydroxybenzimidazole, le 2-méthyl 4-aminobenzimidazole, le 5,6-dihydroxybenzimidazole, le 5-hydroxy 6-méthoxybenzimidazole, le 4,7-dihydroxybenzimidazole, le 4,7-dihydroxy 1-méthylbenzimidazole, le 4,7-diméthoxybenzimidazole, le 5,6-dihydroxy 1-méthylbenzimidazole, le 5,6-dihydroxy 2-méthylbenzimidazole, le 5,6-diméthoxybenzimidazole.

3. Composition tinctoriale pour fibres kératiniques, caractérisée par le fait qu'elle contient dans un milieu approprié pour la teinture des fibres kératiniques et en particulier des cheveux, au moins un précurseur de colorant d'oxydation para et/ou ortho, au moins une métaphénylènediamine et au moins à titre de coupleur un dérivé de benzimidazole répondant à la formule (I) : 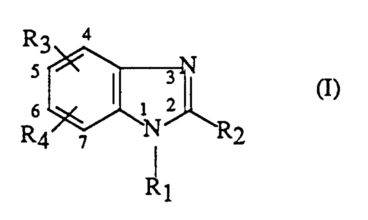 dans laquelle :
R₁ représente un atome d'hydrogène ou un radical alkyle en C₁-C₄ ;
R₂ représente un atome d'hydrogène ou un radical alkyle en C₁-C₄ ou un radical phényle;
R₃ représente un radical hydroxyle, amino ou méthoxy;
R₄ représente un atome d'hydrogène, un radical hydroxyle ou méthoxy ou alkyle en C₁-C₄;
sous réserve que :
- lorsque R₃ désigne NH₂, il occupe la position 4;
- lorsque R₃ est en position 4, R₄ est en position 7;
- lorsque R₃ est en position 5, alors R₄ est en position 6.

4. Composition selon la revendication 3, caractérisée par le fait que le dérivé de benzimidazole de formule (I) est choisi parmi les composés définis dans la revendication 2.

5. Composition selon la revendication 3 ou 4, caractérisée par le fait que les précurseurs de colorants d'oxydation de type para et/ou ortho sont choisis parmi les paraphénylènediamines, les paraaminophénols, les précurseurs hétérocycliques para et les bases dites "doubles" de la famille des bis-phénylalkylènediamines.

6. Composition selon l'une quelconque des revendications 3 à 5, caractérisée par le fait que la paraphénylènediamine est choisie parmi les composés de formule (II) : 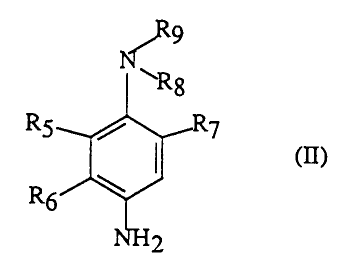 dans laquelle :
R₅, R₆ et R₇, identiques ou différents, représentent un atome d'hydrogène ou d'halogène, un radical alkyle ou hydroxyalkyle ayant 1 à 4 atomes de carbone, un radical alcoxy ayant 1 à 4 atomes de carbone, un radical carboxy ou sulfo;
R₈ et R₉, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ces groupes alkyle ou alcoxy ayant de 1 à 4 atomes de carbone, ou bien R₈ et R₉ forment, conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés.

7. Composition selon l'une quelconque des revendications 3 à 6, caractérisée par le fait que les paraphénylènediamines sont choisies parmi la p-phénylènediamine, la p-toluylènediamine, la méthoxyparaphénylènediamine, la chloroparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl) aniline, la 3-chloro 4-amino N,N-di-(β-hydroxyéthyl)aniline, la 4-amino N,N-(éthylcarbamylméthyl)aniline, la 3-méthyl 4-amino N,N-(éthylcarbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl, β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl) aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl]morpholine, la N-[(4'-amino) phényl]pipéridine, la 2-hydroxyéthylparaphénylène diamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine.

8. Composition selon l'une quelconque des revendications 3 à 5, caractérisée par le fait que les p-aminophénols sont choisies parmi le p-aminophénol, le 2-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol, le 2-β-hydroxyéthylaminométhyl 4-aminophénol.

9. Composition selon l'une quelconque des revendications 3 à 5, caractérisée par le fait que les colorants d'oxydation de type ortho sont choisis parmi lers orthoaminophénols, les orthophénylènediamines.

10. Composition selon l'une quelconque des revendications 3 à 5, caractérisée par le fait que les bases dites doubles de la famille des bis-phénylalkylènediamines répondent à la formule (III) : 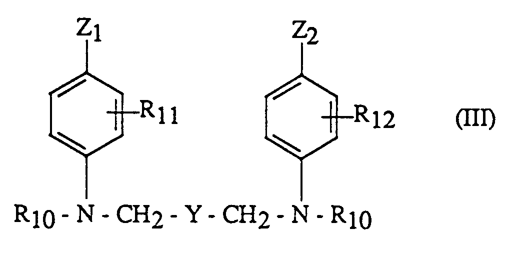 dans laquelle :
Z₁ et Z₂, identiques ou différents, représentent des groupements hydroxyle ou NHR₃, où R₃ désigne un atome d'hydrogène ou un radical alcoyle inférieur;
R₁₁ et R₁₂, identiques ou différents, représentent soit des atomes d'hydrogène, soit des atomes d'halogène, soit encore des groupements alcoyle;
R₁₀ représente un atome d'hydrogène, un groupe alcoyle, hydroxyalcoyle ou aminoalcoyle, dont le reste amino peut être substitué;
Y représente un radical pris dans le groupe constitué par les radicaux suivants : -(CH₂)ₙ-, (CH₂)ₘ-O-(CH₂)ₘ-, -(CH₂)ₘ-CHOH-(CH₂)ₘ-, 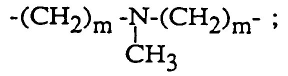
n étant un nombre entier compris entre 0 et 8 et m un nombre entier compris entre 0 et 4, cette base pouvant se présenter sous forme de ses sels d'addition avec des acides.

11. Composition selon la revendication 10, caractérisée par le fait que les bis-phénylalkylènediamines sont choisies parmi le N,N'-bis-(β-hydroxyéthyl)N,N'-bis-(4'-aminophényl)1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N, N'-bis-(4-méthylaminophényl)tétraméthylènediamine, la N,N'-bis-(éthyl)N,N'-bis-(4'-amino 3'-méthylphényl)éthylènediamine.

12. Composition selon l'une quelconque des revendications 3 à 11, caractérisée par le fait que la métaphénylènediamine est choisie parmi les composés répondant à la formule : 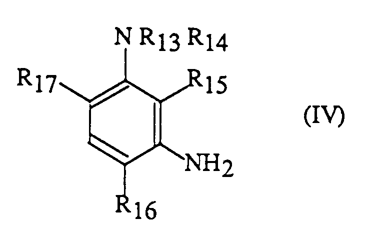 dans laquelle :
R₁₃ R₁₄ désignent, indépendamment l'un de l'autre, hydrogène, un groupement alkyle en C₁-C₄, hydroxyalkyle en C₁-C₄ ;
R₁₅ représente hydrogène, alkyle ou alcoxy en C₁-C₄ ;
R₁₆ représente un atome d'hydrogène ou un groupement alkyl(C₁-C₄) ou hydroxyalcoxy en C₁-C₄ ou alcoxy en C₁-C₄ ;
R₁₇ représente un atome d'hydrogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, halogène, carboxyalcoxy en C₁-C₄, 2',4'-diaminophénoxyalcoxy en C₁-C₄, aminoalcoxy en C₁-C₄ ;
sous réserve que si R₁₇ désigne carboxyalcoxy en C₁-C₄ ou 2',4'-diaminophénoxyalcoxy en C₁-C₄, alors R₁₃, R₁₄, R₁₅ et R₁₆ désignent hydrogène.

13. Composition selon la revendication 12, caractérisée par le fait que les métaphénylènediamines sont choisies parmi le 1,3-diaminobenzène, le 1-méthoxy 2,4-diaminobenzène, le 1-(2-hydroxyéthyloxy)2,4-diaminobenzène, le 1-éthoxy 2,4-diaminobenzène, le 1-méthyl 2,6-diaminobenzène, le 1-amino 3-[(N,N-bis-(2-hydroxyéthyl)amino]benzène, le 1-β-hydroxyéthyl 2,4-diaminobenzène, le 1-méthoxy 2-amino 4-β-hydroxyéthylaminobenzène, le 1,3-diamino 4-β-aminoéthyloxybenzène, le 1-(2-hydroxyéthyloxy)2-amino 4-méthylaminobenzène, l'acide 2,4-diaminophénoxyacétique, le 4,6-bis-(2-hydroxyéthyloxy)1,3-diaminobenzène, le 2,4-diamino 5-méthylphénétol, le 2,4-diamino 5-(β-hydroxyéthyloxy)toluène, le 2,4-diméthoxy 1,3-diaminobenzène.

14. Composition selon l'une quelconque des revendications 3 à 13, caractérisée par le fait qu'elle contient au moins un dérivé de benzimidazole répondant à la formule (I), au moins une paraphénylènediamine, au moins une métaphénylènediamine et éventuellement une base dite double de la famille des bis-phénylalkylènediamines.

15. Composition selon l'une quelconque des revendications 1 à 3 et 14, caractérisée par le fait qu'elle contient également des coupleurs différents des métaphénylènediamines et des dérivés de benzimidazole de formule (I), choisis parmi les métadiphénols, les métaaminophénols, les métaacylaminophénols, les métauréidophénols, les métacarbalcoxyaminophénols, les hydroxynaphtols, les coupleurs à groupement méthylène actif, choisis parmi les dérivés β-cétoniques, les pyrazolones, les coupleurs indoliques.

16. Composition selon la revendication 15, caractérisée par le fait que les coupleurs autres que la métaphénylènediamine ou les dérivés de benzimidazole de formule (I), sont choisis parmi le métaaminophénol, le 1,3-dihydroxybenzène, le 1,3-dihydroxy 4-chlorobenzène, l'α-naphtol, la 6-hydroxybenzomorpholine, le 1-méthyl 2-hydroxy 4-aminobenzène, le 1-méthyl 2-hydroxy 4-(2-hydroxyéthyl) aminobenzène, le 1,3-dihydroxy 2-méthylbenzène, le 1-hydroxy 3,4-méthylènedioxybenzène, le 1-(β-hydroxyéthylamino)3,4-méthylène dioxybenzène, le 2-bromo 4,5-méthylènedioxyphénol, le 2-amino 5-acétamidophénol, la 2,6-diaminopyridine, le 6-hydroxyindole, le 7-hydroxyindole, le 7-aminoindole.

17. Composition tinctoriale selon l'une quelconque des revendications 3 à 16, caractérisée par le fait que le dérivé de benzimidazole de formule (I) est présent dans des proportions comprises entre 0,004 et 3,5% en poids par rapport au poids total de la composition et que les métaphénylènediamines sont présentes dans des proportions comprises entre 0,004 et 3,5% en poids par rapport au poids total de la composition, l'ensemble des précurseurs de colorants par oxydation de type para et/ou ortho et des coupleurs étant présent dans des proportions allant de 0,02 à 10% en poids par rapport au poids total de la composition.

18. Procédé de teinture des fibres kératiniques et en particulier des cheveux humains, caractérisé par le fait que l'on applique sur ces fibres au moins un dérivé de benzimidazole de formule (I) : 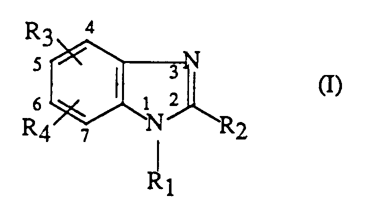 dans laquelle :
R₁ représente un atome d'hydrogène ou un radical alkyle en C₁-C₄ ;
R₂ représente un atome d'hydrogène ou un radical alkyle en C₁-C₄ ou un radical phényle;
R₃ représente un radical hydroxyle, amino ou méthoxy;
R₄ représente un atome d'hydrogène, un radical hydroxyle ou méthoxy ou alkyle en C₁-C₄;
sous réserve que :
- lorsque R₃ désigne NH₂, il occupe la position 4;
- lorsque R₃ est en position 4, R₄ est en position 7;
- lorsque R₃ est en position 5, alors R₄ est en position 6,
au moins un précurseur de colorant d'oxydation para et/ou ortho et au moins une méthaphénylènediamine, chacun de ces composés étant dans un milieu approprié pour la teinture et au moins un agent oxydant dans des quantités suffisantes pour développer une coloration au niveau de la fibre.

19. Composition tinctoriale pour fibres kératiniques, en particulier pour cheveux, prête à l'emploi, caractérisée par le fait qu'elle est telle que définie dans l'une quelconque des revendications 3 à 17 et qu'elle contient en plus un agent oxydant dans des quantités suffisantes pour développer une coloration au niveau des fibres traitées.

20. Procédé de coloration des fibres kératiniques, en particulier des cheveux humains, caractérisé par le fait que l'on applique sur ces fibres une composition telle que définie dans l'une quelconque des revendications 3 à 17, et qu'avant, pendant ou après l'application, on applique sur les fibres une composition contenant un agent oxydant susceptible de développer la coloration au contact des fibres traitées.

## Claims

1. Use of benzimidazole derivatives as couplers for the oxidation dyeing of keratinous fibres, and especially human hair, in the presence of at least one meta-phenylenediamine and at least one para and/or ortho oxidation dye precursor, the benzimidazole derivative corresponding to the formula: 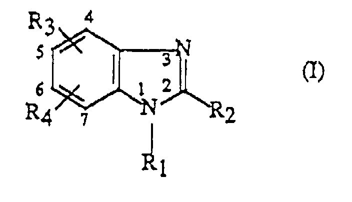 in which:
R₁ represents a hydrogen atom or a C₁-C₄ alkyl radical;
R₂ represents a hydrogen atom or a C₁-C₄ alkyl radical or a phenyl radical;
R₃ represents a hydroxyl, amino or methoxy radical;
R₄ represents a hydrogen atom or a hydroxyl or methoxy or C₁-C₄ alkyl radical;
with the proviso that:
- when R₃ denotes NH₂, it occupies position 4;
- when R₃ is at position 4, R₄ is at position 7;
- when R₃ is at position 5, then R₄ is at position 6.

2. Use according to Claim 1, characterized in that the benzimidazole derivative is chosen from 4-hydroxybenzimidazole, 4-aminobenzimidazole, 4-hydroxy-7-methylbenzimidazole, 2-methyl-4-hydroxybenzimidazole, 1-butyl-4-hydroxybenzimidazole, 2-methyl-4-aminobenzimidazole, 5,6-dihydroxybenzimidazole, 5-hydroxy-6-methoxybenzimidazole, 4,7-dihydroxybenzimidazole, 4,7-dihydroxy-1-methylbenzimidazole, 4,7-dimethoxybenzimidazole, 5,6-dihydroxy-1-methylbenzimidazole, 5,6-dihydroxy-2-methylbenzimidazole and 5,6-dimethoxybenzimidazole.

3. Dyeing composition for keratinous fibres, characterized in that it contains, in a medium suitable for the dyeing of keratinous fibres, and especially hair, at least one para and/or ortho oxidation dye precursor, at least one meta-phenylenediamine and at least, as coupler, one benzimidazole derivative corresponding to the formula (I): 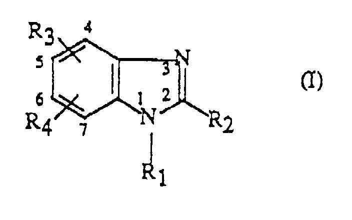 in which:
R₁ represents a hydrogen atom or a C₁-C₄ alkyl radical;
R₂ represents a hydrogen atom or a C₁-C₄ alkyl radical or a phenyl radical;
R₃ represents a hydroxyl, amino or methoxy radical;
R₄ represents a hydrogen atom or a hydroxyl or methoxy or C₁-C₄ alkyl radical;
with the proviso that:
- when R₃ denotes NH₂, it occupies position 4;
- when R₃ is at position 4, R₄ is at position 7;
- when R₃ is at position 5, then R₄ is at position 6.

4. Composition according to Claim 3, characterized in that the benzimidazole derivative of formula (I) is chosen from the compounds defined in Claim 2.

5. Composition according to Claim 3 or 4, characterized in that the para and/or ortho type oxidation dye precursors are chosen from para-phenylenediamines, para-aminophenols, heterocyclic para precursors and so-called "double" bases of the bis(phenyl)alkylenediamine family.

6. Composition according to any one of Claims 3 to 5, characterized in that the para-phenylenediamine is chosen from the compounds of formula (II): 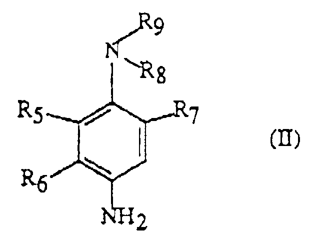 in which:
R₅, R₆ and R₇, which may be identical or different, represent a hydrogen or halogen atom, an alkyl or hydroxyalkyl radical having 1 to 4 carbon atoms, an alkoxy radical having 1 to 4 carbon atoms or a carboxyl or sulpho radical;
R₈ and R₉, which may be identical or different, represent a hydrogen atom or an alkyl, hydroxyalkyl, alkoxyalkyl, carbamoylalkyl, mesylaminoalkyl, acetylaminoalkyl, ureidoalkyl, carbalkoxyaminoalkyl, sulphoalkyl, piperidinoalkyl or morpholinoalkyl radical, these alkyl or alkoxy groups having from 1 to 4 carbon atoms, or alternatively R₈ and R₉, together with the nitrogen atom to which they are linked, form a piperidino or morpholino heterocycle, with the proviso that R₅ or R₇ represents a hydrogen atom when R₈ and R₉ do not represent a hydrogen atom, as well as the salts of these compounds.

7. Composition according to any one of Claims 3 to 6, characterized in that the para-phenylenediamines are chosen from p-phenylenediamine, p-toluylenediamine, methoxy-para-phenylenediamine, chloro-para-phenylenediamine, 2,6-dimethyl-para-phenylenediamine, 2,6-diethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, N,N-diethyl-para-phenylenediamine, N,N-dipropyl-para-phenylenediamine, 3-methyl-4-amino-N,N-diethylaniline, N,N-bis(β-hydroxyethyl)-para-phenylenediamine, 3-methyl-4-amino-N,N-bis(β-hydroxyethyl)aniline, 3-chloro-4-amino-N,N-bis(β-hydroxyethyl)aniline, 4-amino-N-ethyl-N-(carbamoylmethyl)aniline, 3-methyl-4-amino-N-ethyl-N-(carbamoylmethyl)aniline, 4-amino-N-ethyl-N-(β-piperidinoethyl)aniline, 3-methyl-4-amino-N-ethyl-N-(β-piperidinoethyl)aniline, 4-amino-N-ethyl-N-(β-morpholinoethyl)aniline, 3-methyl-4-amino-N-ethyl-N-(β-morpholinoethyl)aniline, 4-amino-N-ethyl-N-(β-acetylaminoethyl)aniline, 4-amino-N-(β-methoxyethyl)aniline, 3-methyl-4-amino-N-ethyl-N-(β-acetylaminoethyl)aniline, 4-amino-N-ethyl-N-(β-mesylaminoethyl)aniline, 3-methyl-4-amino-N-ethyl-N-(β-mesylaminoethyl)aniline, 4-amino-N-ethyl-N-(β-sulphoethyl)aniline, 3-methyl-4-amino-N-ethyl-N-(β-sulphoethyl)aniline, N-(4-aminophenyl)morpholine, N-(4-aminophenyl)piperidine, 2-hydroxyethyl-para-phenylenediamine, fluoro-para-phenylenediamine, carboxy-para-phenylenediamine, sulpho-para-phenylenediamine, 2-isopropyl-para-phenylenediamine, 2-n-propyl-para-phenylenediamine, hydroxy-2-n-propyl-para-phenylenediamine and 2-hydroxymethyl-para-phenylenediamine.

8. Composition according to any one of Claims 3 to 5, characterized in that the p-aminophenols are chosen from p-aminophenol, 2-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol, 2-aminomethyl-4-aminophenol and 2-(β-hydroxyethylaminomethyl)-4-aminophenol.

9. Composition according to any one of Claims 3 to 5, characterized in that the ortho type oxidation dyes are chosen from ortho-aminophenols and ortho-phenylenediamines.

10. Composition according to any one of Claims 3 to 5, characterized in that the so-called double bases of the bis(phenyl)alkylenediamine family correspond to the formula (III): 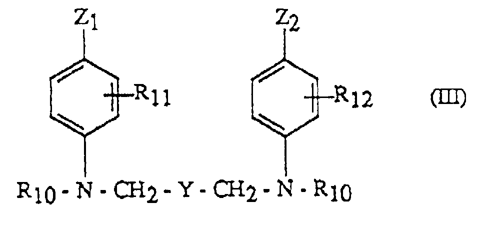 in which:
Z₁ and Z₂, which may be identical or different, represent hydroxyl groups or groups NHR₃, where R₃ denotes a hydrogen atom or a lower alkyl radical;
R₁₁ and R₁₂, which may be identical or different, represent either hydrogen atoms or halogen atoms or alternatively alkyl groups;
R₁₀ represents a hydrogen atom, an alkyl or hydroxyalkyl group or an aminoalkyl group in which the amino residue may be substituted;
Y represents a radical selected from the group consisting of the following radicals: -(CH₂)ₙ-, -(CH₂)ₘ-O-(CH₂)ₘ-, (CH₂)ₘ-CHOH-(CH₂)ₘ-, 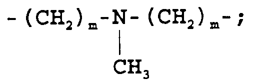
n being an integer between 0 and 8 and m an integer between 0 and 4, it being possible for this base to take the form of its addition salts with acids.

11. Composition according to Claim 10, characterized in that the bis(phenyl)alkylenediamines are chosen from N,N'-bis(β-hydroxyethyl)-N,N'-bis(4-aminophenyl)-1,3-diamino-2-propanol, N,N'-bis(β-hydroxyethyl)-N,N'-bis(4-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4-amino-3-methylphenyl)ethylenediamine.

12. Composition according to any one of Claims 3 to 11, characterized in that the meta-phenylenediamine is chosen from the compounds corresponding to the formula: 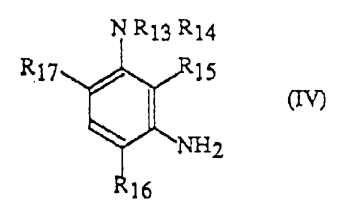 in which:
R₁₃ and R₁₄ denote, independently of one another, hydrogen or a C₁-C₄ alkyl or C₁-C₄ hydroxyalkyl group;
R₁₅ represents hydrogen or C₁-C₄ alkyl or alkoxy;
R₁₆ denotes a hydrogen atom or a C₁-C₄ alkyl or C₁-C₄ hydroxyalkoxy or C₁-C₄ alkoxy group;
R₁₇ represents a hydrogen atom or a C₁-C₄ alkyl, C₁-C₄ alkoxy, C₁-C₄ hydroxyalkyl, C₁-C₄ hydroxyalkoxy, C₂-C₄ polyhydroxyalkoxy, halogen, carboxy(C₁-C₄ alkoxy), 2,4-diaminophenoxy(C₁-C₄ alkoxy) or C₁-C₄ aminoalkoxy group;
with the proviso that, if R₁₇ denotes carboxy(C₁-C₄ alkoxy) or 2,4-diaminophenoxy(C₁-C₄ alkoxy), then R₁₃, R₁₄, R₁₅ and R₁₆ denote hydrogen.

13. Composition according to Claim 12, characterized in that the meta-phenylenediamines are chosen from 1,3-diaminobenzene, 1-methoxy-2,4-diaminobenzene, 1-(2-hydroxyethyloxy)-2,4-diaminobenzene, 1-ethoxy-2,4-diaminobenzene, 1-methyl-2,6-diaminobenzene, 1-amino-3-[N,N-bis(2-hydroxyethyl)amino]benzene, 1-(β-hydroxyethyl)-2,4-diaminobenzene, 1-methoxy-2-amino-4-(β-hydroxyethylamino)benzene, 1,3-diamino-4-(β-aminoethyloxy)benzene, 1-(2-hydroxyethyloxy)-2-amino-4-(methylamino)benzene, 2,4-diaminophenoxyacetic acid, 4,6-bis(2-hydroxyethyloxy)-1,3-diaminobenzene, 2,4-diamino-5-methylphenetole, 2,4-diamino-5-(β-hydroxyethyloxy)toluene and 2,4-dimethoxy-1,3-diaminobenzene.

14. Composition according to any one of Claims 3 to 13, characterized in that it contains at least one benzimidazole derivative corresponding to the formula (I), at least one para-phenylenediamine, at least one meta-phenylenediamine and, where appropriate, a so-called double base of the bis(phenyl)alkylenediamine family.

15. Composition according to any one of Claims 1 to 3 and 14, characterized in that it also contains couplers other than meta-phenylenediamines and the benzimidazole derivatives of the formula (I), chosen from meta-diphenols, meta-aminophenols, meta-acylaminophenols, meta-ureidophenols, meta-carbalkoxyaminophenols, hydroxynaphthols and couplers containing an active methylene group, chosen from β-keto derivatives, pyrazolones and indole couplers.

16. Composition according to Claim 15, characterized in that the couplers other than the meta-phenylenediamine or the benzimidazole derivatives of formula (I) are chosen from meta-aminophenol, 1,3-dihydroxybenzene, 1,3-dihydroxy-4-chlorobenzene, α-naphthol, 6-hydroxybenzomorpholine, 1-methyl-2-hydroxy-4-aminobenzene, 1-methyl-2-hydroxy-4-(2-hydroxyethyl)aminobenzene, 1,3-dihydroxy-2-methylbenzene, 1-hydroxy-3,4-methylenedioxybenzene, 1-(β-hydroxyethylamino)-3,4-methylenedioxybenzene, 2-bromo-4,5-methylenedioxyphenol, 2-amino-5-acetamidophenol, 2,6-diaminopyridine, 6-hydroxyindole, 7-hydroxyindole and 7-aminoindole.

17. Dyeing composition according to any one of Claims 3 to 16, characterized in that the benzimidazole derivative of formula (I) is present in proportions of between 0.004 and 3.5% by weight relative to the total weight of the composition and in that the meta-phenylenediamines are present in proportions of between 0.004 and 3.5% by weight relative to the total weight of the composition, the para and/or ortho type oxidation dye precursors and the couplers collectively being present in proportions ranging from 0.02 to 10% by weight relative to the total weight of the composition.

18. Process for dyeing keratinous fibres, and especially human hair, characterized in that at least one benzimidazole derivative of formula (I): 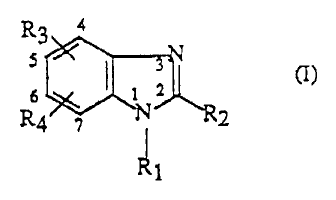 in which:
R₁ represents a hydrogen atom or a C₁-C₄ alkyl radical;
R₂ represents a hydrogen atom or a C₁-C₄ alkyl radical or a phenyl radical;
R₃ represents a hydroxyl, amino or methoxy radical;
R₄ represents a hydrogen atom or a hydroxyl or methoxy or C₁-C₄ alkyl radical;
with the proviso that:
- when R₃ denotes NH₂, it occupies position 4;
- when R₃ is at position 4, R₄ is at position 7;
- when R₃ is at position 5, then R₄ is at position 6,
at least one para and/or ortho oxidation dye precursor and at least one meta-phenylenediamine are applied to these fibres, each of these compounds being in a medium suitable for dyeing, and at least one oxidizing agent in sufficient amounts to develop a coloration on the fibre.

19. Ready-for-use dyeing composition for keratinous fibres, especially for hair, characterized in that it is as defined in any one of Claims 3 to 17 and in that it contains, in addition, an oxidizing agent in sufficient amounts to develop a coloration on the treated fibres.

20. Process for dyeing keratinous fibres, especially human hair, characterized in that a composition as defined in any one of Claims 3 to 17 is applied to these fibres, and in that, before, during or after the application, a composition containing an oxidizing agent capable of developing the coloration on contact with the treated fibres is applied to the fibres.

## Patentansprüche

1. Verwendung von Benzimidazolderivaten als Kuppler zur Oxidationsfärbung keratinischer Fasern und insbesondere der menschlichen Haare, in Gegenwart mindestens eines m-Phenylendiamins und mindestens einer Oxidationsfarbstoff-Vorstufenverbindung vom p- und/oder o-Typ, wobei das Benzimidazolderivat die Formel aufweist: 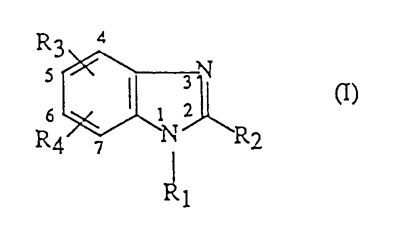 worin gilt:
R₁ stellt ein Wasserstoffatom oder einen C₁₋₄-Alkylrest dar;
R₂ stellt ein Wasserstoffatom oder einen C₁₋₄-Alkyl- oder einen Phenylrest dar;
R₃ stellt einen Hydroxyl-, Amino- oder Methoxyrest dar;
R₄ stellt ein Wasserstoffatom, einen Hydroxyl- oder Methoxy- oder einen C₁₋₄-Alkylrest dar,
mit der Maßgabe, daß gilt:
- wenn R₃ NH₂ bedeutet, besetzt dieser Rest die Position 4;
- wenn R₃ in Position 4 vorliegt, liegt R₄ in Position 7 vor;
- wenn R₃ in Position 5 vorliegt, dann liegt R₄ in Positi on 6 vor.

2. Verwendung gemäß Anspruch 1,
dadurch **gekennzeichnet**, daß
das Benzimidazol aus 4-Hydroxybenzimidazol, 4-Aminobenzimidazol, 4-Hydroxy-7-methylbenzimidazol, 2-Methyl-4-hydroxybenzimidazol, 1-Butyl-4-hydroxybenzimidazol, 2-Methyl-4-aminobenzimidazol, 5,6-Dihydroxybenzimidazol, 5-Hydroxy-6-methoxybenzimidazol, 4,7-Dihydroxybenzimidazol, 4,7-Dihydroxy-1-methylbenzimidazol, 4,7-Dimethoxybenzimidazol, 5,6-Dihydroxy-1-methylbenzimida-zol, 5,6-Dihydroxy-2-methylbenzimidazol und aus 5,6-Dimethoxybenzimidazol ausgewählt ist.

3. Färbezusammensetzung für keratinische Fasern,
dadurch **gekennzeichnet**, daß
sie in einem zur Färbung keratinischer Fasern und insbesondere der Haare geeigneten Milieu mindestens eine Oxidationsfarbstoff-Vorstufenverbindung vom p- und/oder o-Typ, mindestens ein m-Phenylendiamin und mindestens als Kuppler ein Benzimidazolderivat der Formel (I) enthält: 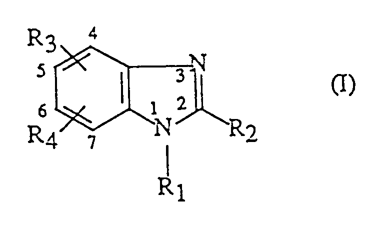 worin gilt:
R₁ stellt ein Wasserstoffatom oder einen C₁₋₄-Alkylrest dar;
R₂ stellt ein Wasserstoffatom oder einen C₁₋₄-Alkyl- oder einen Phenylrest dar;
R₃ stellt einen Hydroxyl-, Amino- oder Methoxyrest dar;
R₄ stellt ein Wasserstoffatom, einen Hydroxyl- oder Methoxy- oder einen C₁₋₄-Alkylrest dar,
mit der Maßgabe, daß gilt:
- wenn R₃ NH₂ bedeutet, besetzt dieser Rest die Position 4;
- wenn R₃ in Position 4 vorliegt, liegt R₄ in Position 7 vor;
- wenn R₃ in Position 5 vorliegt, dann liegt R₄ in Positi on 6 vor.

4. Zusammensetzung gemäß Anspruch 3,
dadurch **gekennzeichnet**, daß
das Benzimidazolderivat der Formel (I) aus den in Anspruch 2 definierten Verbindungen ausgewählt ist.

5. Zusammensetzung gemäß Anspruch 3 oder 4,
dadurch **gekennzeichnet**, daß
die Oxidationsfarbstoff-Vorstufenverbindungen vom p- und/oder o-Typ aus p-Phenylendiaminen, p-Aminophenolen, heterozylischen p-Vorstufenverbindungen und aus als "Doppelte" bezeichneten Basen der Familie der Bisphenylalkylendiamine ausgewählt sind.

6. Zusammensetzung gemäß einem der Ansprüche 3 bis 5,
dadurch **gekennzeichnet**, daß
das p-Phenylendiamin aus Verbindungen der Formel (II): 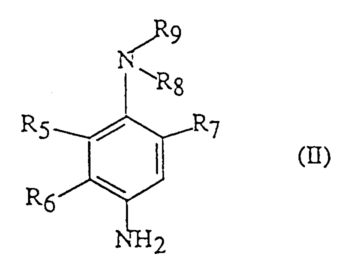 worin gilt:
R₅, R₆ und R₇ stellen, gleich oder verschieden, ein Wasserstoff- oder Halogenatom, eine Alkyl- oder Hydroxyalkylrest mit 1 bis 4 Kohlenstoffatomen, einen Alkoxyrest mit 1 bis 4 Kohlenstoffatomen, einen Carboxy- oder Sulforest dar;
R₈ und R₉ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalcoxyaminoalkyl-, Sufloalkyl-, Piperidinoalkyl- oder einen Morpholinoalkylrest dar, wobei die Alkyl- oder Alkoxygruppen 1 bis 4 Kohlenstoffatome aufweisen, oder R₈ und R₉ bilden, zusammen mit dem Stickstoffatom, an das sie gebunden sind, auch einen Piperidino- oder Morpholino-Heterozyklus, mit der Maßgabe, daß R₅ oder R₇ ein Wasserstoffatom darstellen, wenn R₈ und R₉ kein Wasserstoffatom darstellen,
sowie aus den Salzen dieser Verbindungen ausgewählt ist.

7. Zusammensetzung gemäß einem der Ansprüche 3 bis 6,
dadurch **gekennzeichnet**, daß
die p-Phenylendiamine aus p-Phenylendiamin, p-Toluylendiamin, Methoxy-p-phenylendiamin, Chlor-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl, β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethylanilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin, 2-Isopropyl-p-phenylendiamin, 2-n-Propyl-p-phenylendiamin, Hydroxy-2-n-propyl-p-phenylendiamin und aus 2-Hydroxymethyl-p-phenylendiamin ausgewählt sind.

8. Zusammensetzung gemäß einem der Ansprüche 3 bis 5,
dadurch **gekennzeichnet**, daß
die p-Aminophenole aus p-Aminophenol, 2-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymehtyl-4-aminophenol, 2-Aminomethyl-4-aminophenol und 2-β-Hydroxyethylaminomethyl-4-aminophenol ausgewählt sind.

9. Zusammensetzung gemäß einem der Ansprüche 3 bis 5,
dadurch **gekennzeichnet**, daß
die Oxidationsfarbstoffe vom o-Typ aus o-Aminophenolen und o-Phenylendiaminen ausgewählt sind.

10. Zusammensetzung gemäß einem der Ansprüche 3 bis 5,
dadurch **gekennzeichnet**, daß
die als "Doppelte" bezeichneten Basen der Familie der Bisphenylalkylendiamine die Formel (III) aufweisen: 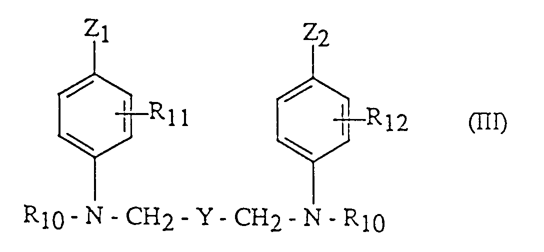 worin gilt:
Z₁ und Z₂ stellen, gleich oder verschieden, Hydroxyl- oder NHR₃-Gruppen dar, worin R₃ ein Wasserstoffatom oder einen Niedrigalkylrest bedeutet;
R₁₁ und R₁₂ stellen, gleich oder verschieden, entweder Wasserstoffatome oder Halogenatome oder auch Alkylgruppen dar;
R₁₀ stellt ein Wasserstoffatom, eine Alkyl-, Hydroxyalkyl- oder Aminoalkylgruppe dar, worin der Aminorest substituiert sein kann;
Y stellt einen Rest aus der Gruppe aus den folgenden Resten dar: -(CH₂)ₙ-, (CH₂)ₘ-O-(CH₂)ₘ-, -(CH₂)ₘ-CHOH-(CH₂)ₘ-, 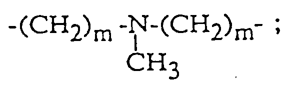
worin n eine ganze Zahl von 0 bis 8 und m eine ganze Zahl von 0 bis 4 sind,
wobei diese Base in Form von Additionssalzen mit Säuren vorliegen kann.

11. Zusammensetzung gemäß Anspruch 10,
dadurch **gekennzeichnet**, daß
die Bisphenylalkylendiamine aus N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin und aus N,N'-Bis(ethyl)-N,N'-bis(4'-amino-3'-methylphenyl)ethylendiamin ausgewählt sind.

12. Zusammensetzung gemäß einem der Ansprüche 3 bis 11,
dadurch **gekennzeichnet**, daß
das m-Phenylendiamin aus Verbindungen der Formel ausgewählt ist: 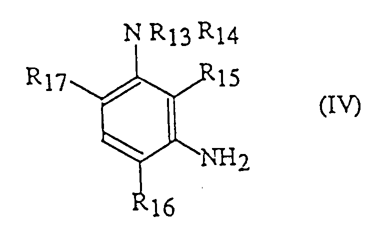 worin gilt:
R₁₃ und R₁₄ bedeuten, unabhängig voneinander, Wasserstoff, eine C₁₋₄-Alkyl- oder eine C₁₋₄-Hydroxyalkylgruppe;
R₁₅ bedeutet Wasserstoff oder eine C₁₄-Alkyl- oder -Alkoxygruppe;
R₁₆ bedeutet ein Wasserstoffatom oder eine C₁₋₄-Alkyl- oder C₁₋₄-Hydroxyalkoxy- oder eine C₁₋₄-Alkoxygruppe;
R₁₇ bedeutet ein Wasserstoffatom oder eine C₁₋₄-Alkyl-, C₁₋₄-Alkoxy-, C₁₋₄-Hydroxyalkyl-, C₁₋₄-Hydroxyalkoxy-, C₂₋₄-Polyhydroxyalkoxy-, ein Halogenatom, einen C₁₋₄-Carboxyalkoxy-, 2',4'-Diaminophenoxy-C₁₋₄-alkoxy- oder einen C₁₋₄-Aminoalkoxyrest,
mit der Maßgabe, daß, wenn R₁₇ einen Carboxyalkoxy- oder 2',4'-Diaminophenoxyalkoxyrest bedeutet, R₁₃, R₁₄, R₁₅ und R₁₆ dann Wasserstoff bedeuten.

13. Zusammensetzung gemäß Anspruch 12,
dadurch **gekennzeichnet**, daß
die m-Phenylendiamine aus 1,3-Diaminobenzol, 1-Methoxy-2,4-diaminobenzol, 1-(2-Hydroxyethyloxy)-2,4-diaminobenzol, 1-Ethoxy-2,4-diaminobenzol, 1-Methyl-2,6-diaminobenzol, 1-Amino-3-((N,N-bis(2-hydroxyethyl)amino)benzol, 1-β-Hydroxyethyl-2,4-diaminobenzol, 1-Methoxy-2-amino-4-β-hydroxyethylaminobenzol, 1,3-Diamino-4-β-aminoethyloxybenzol, 1-(2-Hydroxyethyloxy)-2-amino-4-methylaminobenzol, 2,4-Diaminophenoxyessigsäure, 4,6-Bis(2-hydroxyethyloxy)-1,3-diaminobenzol, 2,4-Diamino-5-methylphenol, 2,4-Diamino-5-(β-hydroxyethyloxy)toluol und aus 2,4-Dimethoxy-1,3-diaminobenzol ausgewählt sind.

14. Zusammensetzung gemäß einem der Ansprüche 3 bis 13,
dadurch **gekennzeichnet**, daß
sie mindestens ein Benzimidazolderivat der Formel (I), mindestens ein p-Phenylendiamin, mindestens ein m-Phenylendiamin und gegebenenfalls mindestens eine als "Doppelte" bezeichnete Base der Familie der Bisphenylalkylendiamine enthält.

15. Zusammensetzung gemäß einem der Ansprüche 3 und 14,
dadurch **gekennzeichnet**, daß
sie auch Kuppler enthält, die sich von m-Phenylendiaminen und den Benzimidazolderivaten der Formel (I) unterscheiden und aus m-Diphenolen, m-Aminophenolen, m-Acylaminophenolen, m-Ureidophenolen, m-Carbalkoxyaminophenolen, Hydroxynaphtholen und aus Kupplern mit aktiver Methylengruppe ausgewählt sind, die wiederum aus β-Ketoderivaten, Pyrazolonen und aus Indol-Kupplern ausgewählt sind.

16. Zusammensetzung gemäß Anspruch 15,
dadurch **gekennzeichnet**, daß
die vom m-Phenylendiamin oder den Benzimidazolderivaten der Formel (I) unterschiedenen Kuppler aus m-Aminophenol, 1,3-Dihydroxy-4-chlorbenzol, 1,3-Dihydroxybenzol, α-Naphthol, 6-Hydroxybenzomorpholin, 1-Methyl-2-hydroxy-4-aminobenzol, 1-Methyl-2-hydroxy-4-(2-hydroxyethyl)aminobenzol, 1,3-Dihydroxy-2-methylbenzol, 1-Hydroxy-3,4-methylendioxybenzol, 1-(β-Hydroxyethylamino)-3,4-methylendioxybenzol, 2-Brom-4,5-methylendioxyphenol, 2-Amino-5-acetamidophenol, 2,6-Diaminopyridin, 6-Hydroxyindol, 7-Hydroxyindol und 7-Aminoindol ausgewählt sind.

17. Färbezusammensetzung gemäß einem der Ansprüche 3 bis 16,
dadurch **gekennzeichnet**, daß
das Benzimidazolderivat der Formel (I) in Mengenanteilen von 0,004 bis 3,5 Gew.%, bezogen auf das Gesamtgewicht der Zusammensetzung, und die m-Phenylendiamine in Mengenanteilen von 0,004 bis 3,5 Gew.% vorhanden sind, bezogen auf das Gesamtgewicht der Zusammensetzung, wobei die Gesamtheit der Oxidationsfarbstoff-Vorstufenverbindungen vom p- und/oder o-Typ und der Kuppler in Mengenanteilen von 0,02 bis 10 Gew.% vorliegen, bezogen auf das Gesamtgewicht der Zusammensetzung.

18. Verfahren zur Färbung keratinischer Fasern und insbesondere der menschlichen Haare,
dadurch **gekennzeichnet**, daß
man auf diese Fasern mindestens ein Benzimidazolderivat der Formel (I): 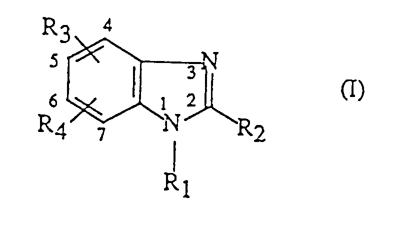 worin gilt:
R₁ stellt ein Wasserstoffatom oder einen C₁₋₄-Alkylrest dar;
R₂ stellt ein Wasserstoffatom oder einen C₁₋₄-Alkyl- oder einen Phenylrest dar;
R₃ stellt einen Hydroxyl-, Amino- oder Methoxyrest dar;
R₄ stellt ein Wasserstoffatom, einen Hydroxyl- oder Methoxy- oder einen C₁₋₄-Alkylrest dar,
mit der Maßgabe, daß gilt:
- wenn R₃ NH₂ bedeutet, besetzt dieser Rest die Position 4;
- wenn R₃ in Position 4 vorliegt, liegt R₄ in Position 7 vor,
- wenn R₃ in Position 5 vorliegt, dann liegt R₄ in Positi on 6 vor,
mindestens eine Oxidationsfarbstoff-Vorstufenverbindung vom p- und/oder o-Typ und mindestens ein m-Phenylendiamin, wobei jede dieser Verbindungen in einem zur Färbung geeigneten Milieu vorliegt, und mindestens ein oxidierendes Mittel in Mengen aufbringt, die ausreichen, um eine Färbung auf Faserniveau zu entwicklen.

19. Färbezusammensetzung für keratinische Fasern, insbesondere für Haare, die gebrauchsfertig vorliegt und dadurch gekennzeichnet ist, daß sie eine der in jedem der Ansprüche 3 bis 17 definierte ist und zusätzlich ein oxidierendes Mittel in Mengen enthält, die ausreichen, um eine Färbung auf den behandelten Fasern zu entwickeln.

20. Verfahren zur Färbung keratinischer Fasern, insbesondere der menschlichen Haare,
dadurch **gekennzeichnet**, daß
man auf diese Fasern eine in jedem der Ansprüche 3 bis 17 definierte Zusammensetzung und vor, bei oder nach dieser Aufbringung auf die Fasern eine Zusammensetzung aufträgt, die ein oxidierendes Mittel enthält, das geeignet ist, die Färbung bei Kontakt mit den behandelten Fasern zu entwickeln.
